# EUROPEAN PATENT APPLICATION

(11) **EP 4 226 918 A1**
(43) Date of publication of application: **16.08.2023**
(21) Application number: 22156902.3
(22) Date of filing: 15.02.2022
(51) Int. Cl.: A61K 9/48, A61K 38/06, A61K 38/07, A61K 38/10

(54) **PHARMACEUTICAL SINGLE DOSAGE FORM FOR ORAL DELIVERY OF PEPTIDES**

(71) Applicant: Majewski, Filip, 32-300 Olkusz (PL)
(72) Inventor: Majewski, Filip, 32-300 Olkusz (PL)
(74) Representative: Twardowska-Czerwinska, Aleksandra

(57) **Abstract**

Present invention refers to a pharmaceutical single dosage form for oral delivery consisting of two capsules, an outer enteric capsule, and an inner capsule, wherein said inner capsule comprises at least one physiologically active peptide agent and at least one absorption enhancer, and wherein said outer capsule contains inner capsule and an organic acid.

## Description

### FIELD OF THE INVENTION

The present invention relates to oral peptide pharmaceuticals. Particularly, the present invention relates to a single dosage form comprising a peptide agent and an organic acid wherein the peptide agent and the organic acid are spatially separately in a simple way that enhances stability of the single dosage form, and following oral administration, the peptide agent is delivered to the target part of the digestive system. This enhances the bioavailability of the peptide agent.

### DESCRIPTION OF THE RELATED ART

Numerous peptides are known from their therapeutic use. Therapeutically effective amounts of such biologically relevant peptides may be administered to patients in a variety of ways. However, the most preferred oral administration is very difficult to achieve with this type of compounds.

Peptide pharmaceuticals used in the prior art have been frequently administered by injection or by nasal administration (e.g., Insulin). Oral administration is problematic as peptide agents are very susceptible to degradation in the environment of the digestive system as in the stomach and intestines. Proteolytic enzymes of both stomach and intestine may degrade peptides, leading to their inactivity before they are absorbed into the bloodstream. Any amount of a peptide that survives proteolytic degradation by proteases of the stomach (typically having acidic pH optima) is later confronted with proteases of the small intestine and enzymes secreted by the pancreas (typically having neutral to basic pH optima). The problem is even more evident when very active peptide pharmaceuticals are administered in a very small amount per a single dosage form. In such cases, protection of the pharmaceuticals becomes a key issue.

The prior art teaches how to overcome the problem with degradation of the peptide agents in the stomach after oral administration.

EP0929270A1 discloses a pharmaceutical composition for oral delivery of a physiologically active peptide agent comprising: (A) a therapeutically effective amount of said active peptide; (B) at least one pharmaceutically acceptable pH lowering agent; (C) at least one absorption enhancer effective to promote bioavailability of said active agent; and (D) an acid resistant protective vehicle effective to transport said pharmaceutical composition through the stomach of a patient while preventing contact between said active peptide agent and stomach proteases. The pH-lowering compound to be administered with each administration of peptide should preferably be an amount which, when it is released into the intestine, is sufficient to lower the local intestinal pH substantially below the pH optima for proteases found there. Although the peptide agent is protected against the stomach environment, the active peptide agent is intermixed with pH lowering agent and is directly influenced by said pH lowering agent in the intestine, where the protective vehicle (barrier) dissolves. pH lowering agents are preferably organic acids. Therefore, such a pharmaceutical composition has a lower stability due to a constant contact with the pH lowering agents, especially an acid, during storage.

Further disclosures tried to tackle the stability issue by several approaches.

US2003017203A1 discloses a pharmaceutical composition for oral delivery of a peptide comprising: a lamination having a first layer comprising at least one pharmaceutically acceptable pH-lowering agent and a second layer comprising a therapeutically effective amount of a peptide; at least one absorption enhancer effective to promote bioavailability of said peptide; and an enteric coating surrounding said lamination. The tablet is prepared by spray fluid bed process which means that homogeneous distribution of the polypeptide onto a massive amount of excipients is necessary. When for manufacturing of the layered tablet a rotary press is used, the peptide is mixed in binder solution. Both spray fluid process and layered tablet manufacturing require special expensive equipment. A necessity for spraying a peptide solution on a fluid bed, especially in case of very active, low-dose peptides, leads to deterioration of the peptide affecting its stability. Furthermore, tablet manufacturing process generally involves also a significant mechanical stress that further diminishes the stability of the peptide.

EP2152195B1 discloses a pharmaceutical composition for oral delivery of a physiologically active peptide agent comprising said active peptide agent intermixed with pharmaceutically acceptable acid particles that are coated with a pharmaceutically acceptable protective coating that is non-acidic and has a solubility in water of at least one gram per 100 millilitres of water at room temperature to separate the acid particles from the active peptide agent. This approach requires the use of a complex process to obtain coated particles of the acid component and special equipment. Depending on the acid used, it is necessary to adapt a suitable protective coating, which must have solubility in water and be inert to the peptide with which it is in contact.

There is still a need in the art to provide a new peptide delivery system which overcome disadvantages. Especially, there is a need to provide a very simple and cost-efficient system that will be versatile for both known and future peptide agents. The present invention solves these problems.

### SUMMARY OF THE INVENTION

The object of the invention is to provide a peptide delivery system in the form of a single dosage form, that is simple to manufacture, better prevents against undesirable effects of acids on the peptides and maintains good dissolution profile. Furthermore, all the ingredients of the single dosage form are released in the intestines in a close time proximity, thus enhancing bioavailability.

The further object of the invention is to simplify manufacture process of the single dosage form by avoiding tableting process (compression), as well as other processes such as fluid-bed processing, drying, wet granulation, spray-granulation, milling, etc. that may cause unpreferable contact of the peptide agent with water and other factors such as elevated temperature, mechanical stress etc.

The single dosage form of the invention, in contrary to tablets, avoids the need for use of vehicles, carriers, lubricants, and other fillers which have to be co-processed with peptides what decreases its stability.

It was surprisingly found that the object of the invention is realized by a single dosage form for oral delivery of physiologically active peptide agents of the invention.

A subject matter of the invention is a pharmaceutical single dosage form for oral delivery consisting of two capsules, an outer enteric capsule, and an inner capsule, wherein said inner capsule comprises at least one physiologically active peptide agent and at least one absorption enhancer, and wherein said outer capsule contains inner capsule and an organic acid.

The peptide agent is preferably composed of 3-20 amino acids, preferably 3-16. More preferably the peptide agent is selected from GHK-Cu, Epithalon, ARG-BPC-157.

Preferably the inner capsule is enteric capsule or non-enteric capsule.

Preferably the absorption enhancer is selected from the group comprises (a) salicylates such as sodium salicylate, 3-methoxysalicylate, 5-methoxysalicylate and homovanilate; (b) bile acids such as taurocholic, tauorodeoxycholic, deoxycholic, cholic, glycholic, lithocholate, chenodeoxycholic, ursodeoxycholic, ursocholic, dehydrocholic, fusidic, etc.; (c) non-ionic surfactants such as polyoxyethylene ethers (e.g. Brij 36T, Brij 52, Brij 56, Brij 76, Brij 96, Texaphor A6, Texaphor A14, Texaphor A60 etc.), p-t-octyl phenol polyoxyethylenes (Triton X-45, Triton X-100, Triton X-114, Triton X-305 etc.) nonylphenoxypoloxyethylenes (e.g. Igepal CO series), polyoxyethylene sorbitan esters (e.g. Tween-20, Tween-80 etc.); (d) anionic surfactants such as dioctyl sodium sulfosuccinate; (e) lyso-phospholipids such as lysolecithin and lysophosphatidylethanolamine; (f) acylcarnitines, acylcholines and acyl amino acids such as lauroylcarnitine, myristoylcarnitine, palmitoylcarnitine, lauroylcholine, myristoylcholine, palmitoylcholine, hexadecyllysine, N-acylphenylalanine, N-acylglycine etc.; g) water soluble phospholipids such as diheptanoylphosphatidylcholine, dioctylphosphatidylcholine etc.; (h) medium-chain glycerides which are mixtures of mono-, di- and triglycerides containing medium-chain-length fatty acids (caprylic, capric and lauric acids); (i) ethylene-diaminetetraacetic acid; (j) cationic surfactants such as cetylpyridinium chloride; (k) fatty acid derivatives of polyethylene glycol such as Labrasol, Labrafac, etc.; and (I) alkylsaccharides such as lauryl maltoside, lauroyl sucrose, myristoyl sucrose, palmitoyl sucrose; cationic ion exchange agents (e.g. surface-active agent) such as protamine chloride or any other polycation, or their mixtures. Preferably the absorption enhancer is acyl L-carnitine, more preferably lauroyl L-carnitine.

Preferably organic acid is selected from the group comprises carboxylic acids such as acetylsalicylic, acetic, ascorbic, citric, fumaric, glucuronic, glutaric, glyoxylic, isocitric, isovaleric, lactic, maleic, oxaloacetic, propionic, pyruvic, succinic, tartaric, valeric, and the like, amino acids and salts thereof (e.g. amino acid hydrochlorides) or derivatives thereof such as acetylglutamic acid, alanine, arginine, asparagine, aspartic acid, betaine, carnitine, carnosine, citrulline, creatine, glutamic acid, glycine, histidine, hydroxylysine, hydroxyproline, hypotaurine, isoleucine, leucine, lysine, methylhistidine, norleucine, ornithine, phenylalanine, proline, sarcosine, serine, taurine, threonine, tryptophan, tyrosine, valine, or their mixtures. Preferably the organic acid is citric acid.

The amount of peptide agent is preferably 0.2 mg - 20 mg, the amount of absorption enhancer is 50 mg - 150 mg and the amount of organic acid is 200 mg - 500 mg per single dosage form.

In accordance with the invention, patients that need treatment with peptide agents (active ingredients) are provided with an oral pharmaceutical dosage form. When the single dosage form of the invention is administered to a patient, the organic acid is released in the small intestine in a close time proximity with release of the peptide agent and the absorption enhancer. This reduces the activity of neutral to basic-acting proteases (e.g., luminal or digestive protease and proteases of the brush border membrane) by lowering pH below the optimal activity range of these proteases. Thus, the peptide active agents are less vulnerable to proteolytic degradation and can be successfully transported into the bloodstream.

Without intending to be bound by theory, the materials and structure of the single dosage form of the present invention reduces adverse interactions between the organic acid and the other components of the single dosage form. It is further believed that the invention herein provides, at least, not comparable, or higher bioavailability of the peptide agents in comparison to delivery systems known in the prior art e.g., when acid particles are coated with protective coating. The bioavailability of the peptide delivered by the single dosage form is better than by a simple dosage form when a powdered acid is intermixed with the peptide agent, what was proven below by appropriate examples.

### BRIEF DESCRIPTION OF THE DRAWINGS

Fig. 1 is a graph of overlapped chromatograms for GHK-Cu for inventive single dosage form and two comparative dosage forms showing concentration of peptide agent after 120 minutes from taking dosages.
Fig. 2 presents a comparison of the pharmacokinetic profiles of a peptide GHK-Cu for inventive single dosage form and two comparative dosages.
Fig. 3 is a graph of overlapped chromatograms for Epithalon for inventive single dosage form and two comparative dosage forms showing concentration of peptide agent after 120 minutes from taking dosages.
Fig. 4 presents a comparison of the pharmacokinetic profiles of a peptide Epithalon for inventive single dosage form and two comparative dosages.
Fig. 5 is a graph of overlapped chromatograms for Arg-BPC-157 for inventive single dosage form and two comparative dosage forms showing concentration of peptide agent after 120 minutes from taking dosages.
Fig. 6 presents a comparison of the pharmacokinetic profiles of a peptide Arg-BPC-157 for inventive single dosage form and two comparative dosages.
Fig. 7 is a sectional view of a pharmaceutical single dosage form of the invention. Shown inner and outer capsules are not necessarily to scale and is only for the purpose of illustrating preferred relative locations of components of the single dosage form. The preferred amounts of material used in the various layers are discussed elsewhere herein.

### DETAILED DESCRIPTION OF THE INVENTION

In accordance with the invention, patients in need of treatment with peptide active ingredients are provided with the single dosage form. A patient that benefits from the single dosage form is anyone who needs increased levels of a peptide.

The single dosage form of the invention is composed of two capsules of an ordinary size known in the pharmaceutical industry. One of the capsules is an inner capsule and has a smaller size than the outer capsule, wherein the outer capsules contains/encapsules the inner capsule closed in the outer capsule forming a kind of a capsule-in-capsule system.

For example, but without limitation, the inner capsule is of size 5, 4 or 3, and, for example, but without limitation, the outer capsule is of size 0, 00 or 000. Preferably, the inner capsule is of size 5, and the outer capsule is of size 0. Also preferably when inner capsule is of size 4, and the outer capsule is 00. Also preferably when inner capsule is of size 3, and the outer capsule is 000. The person skilled in the art would know how to choose capsules available on the market to be able to put one smaller, inner capsule into the other, bigger, outer capsule so that there is still space in the outer capsule for an organic acid.

The outer capsule must be made of an enteric material. The inner capsule may be made of an enteric or non-enteric material. Capsules made of enteric material and capsules made of non-enteric material are known in the art, and commercially available, for example, capsules available from ADOX Sp. z o. o. (https://kapsulki.com.pl/) or Lonza (https://pharma.lonza.com/offerings/capsule-delivery-solutions).

As used herein, "enteric" relates to a material that does not dissolve or disintegrate in the gastric environment, and it helps to protect active agents, such as peptide agents, from the acidity of the stomach by their release after the stomach (usually in the upper tract of the intestine).

Any enteric capsules that protect the content of the single dosage form against stomach proteases and releases peptide agents in the intestine is suitable. Many enteric capsules are known in the art and are useful in the present invention. Examples include capsules made of cellulose acetate phthalate, hydroxypropylmethylethylcellulose succinate, hydroxypropylmethylcellulose phthalate, polyvinyl acetate phthalate, and methacrylic acid-methyl methacrylate copolymer.

As used herein, "non-enteric" relates to a material that can dissolve or disintegrate in the gastric environment.

Non-enteric capsule may be made of a water-soluble material. Many water-soluble capsules are known in the art and are useful in the present invention. Examples include capsule made of gelatine, hydroxypropylmethylcellulose, hydroxypropylcellulose and methylcellulose.

According to the present invention, the inner capsule comprises at least one physiologically active peptide agent and at least one absorption enhancer.

### Peptides

According to the present invention, all known physiologically active peptides may be used as the peptide agents, components of the single dosage form of the invention, comprised in the inner capsule. Preferably, the peptide agent is a peptide composed of 3-20 amino acids, preferably 3-16 amino acids. Most preferably, the peptide agent is selected from GHK-Cu, Epithalon, or ARG-BPC-157. In the invention, GHK-Cu relates to a tripeptide with the amino acid sequence glycyl-L-histidyl-L-lysine in the form of Cu complex (CAS No 89030-95-5), Epithalon relates to a tetrapeptide with the amino acid sequence Ala-Glu-Asp-Gly (CAS No 307297-39-8), and ARG-BPC-157 relates to an arginine salt of BPC-157 peptide with the amino acid sequence Gly-Glu-Pro-Pro-Pro-Gly-Lys-Pro-Ala-Asp-Asp-Ala-Gly-Leu-Val-Arg, Body Protection Compound (CAS for BPC-157 No 137525-51-0).

Both man-made and natural peptides can be orally delivered in accordance with the invention.

The peptide is to be understood by the person skilled in the art as short chain of amino acids linked by peptide bonds. Simple derivatives of the peptides are also in the scope of the invention. A person skilled in the art would know what simple derivatives are. These are compounds which come under the definition of the peptide known in the art after the transformation into the derivative. The exemplary derivatives are chosen, but not limiting, from the group of metal complexes, N-terminal modified peptides, C-terminal modified peptides, or peptides containing unnatural amino acids.

The metal complexes can be for example copper complexes.

The C-terminal modified peptides can be amides, alkylamides, anilides, aldehydes, or esters.

The N-terminal modified peptides can be N-acylated compound (e.g., acetyl, formyl, pyroglutamyl, fatty acids), ureas, carbamates, sulfonamides, or N-alkylated compounds.

The peptides containing unnatural amino acids can be peptides containing D-amino acids, homoamino acids, N-methyl amino acids, or alpha-methyl amino acids or unusual amino acids such as citrulline or naphtylalanine.

Such modifications are customary in the art, and can be provided by external suppliers such as Pepscan (see, for example, https://www.pepscan.com/custom-peptide-synthesis/peptide-modifications/unusual-non-natural-amino-acids/, https://www.pepscan.com/custom-peptide-synthesis/peptide-modifications/n-terminal-modifications/, https://www.pepscan.com/custom-peptide-synthesis/peptide-modifications/c-terminal-modifications/)

Preferably, the derivatives are compounds prepared by addition of arginine, ornithine, or lysine to the peptides, preferably, N-terminally. Such derivatives can be obtained by general methods known in the art or by external providers such as Pepscan.

Of the invention, the peptide agent can be a single peptide agent or a mixture of different peptide agent.

### Absorption Enhancer

In accordance with the invention an absorption enhancer is comprised in the inner capsule together with the peptide agent.

The enhancer, which may be a solubility enhancer and/or transport enhancer, aids transport of the peptide agent from the intestine to the blood and may promote the process so that it better occurs during the time of reduced intestinal pH and reduced intestinal proteolytic activity. Many surface-active agents may act as both solubility enhancers and transport (uptake) enhancers. Again, without intending to be bound by theory, it is believed that enhancing solubility desirably provides (i) a more simultaneous release of the peptide agent of the invention into the aqueous portion of the intestine, (ii) better solubility of the peptide agent in, and transport through, a mucous layer along the intestinal walls. Once the peptide agent reaches the intestinal walls, an uptake enhancer provides better transport through the brush border membrane of the intestine into the blood, via either transcellular or paracellular transport. As discussed in more detail below, many preferred compounds may provide both functions. In those instances, preferred embodiments utilizing both functions may do so by adding only one additional compound to the pharmaceutical composition. In other embodiments, separate absorption enhancers may provide the two functions separately.

When surface-active agents are used as the absorption enhancers, it is preferred that they are free-flowing powders for facilitating the mixing and loading of capsules during the manufacturing process. Because of inherent characteristics of certain peptides (e.g., their isoelectric point, molecular weight, amino acid composition, etc.), certain surface-active agents interact best with certain peptides. Indeed, some can undesirably interact with the charged portions of certain peptides and thus prevent their absorption, thus undesirably resulting in decreased bioavailability. It is preferred, when trying to increase the bioavailability of peptides that any surface-active agent used as an absorption enhancer be selected from the group consisting of (i) anionic surface-active agents that are cholesterol derivatives (e.g., bile acids), (ii) cationic surface agents (e.g., acyl carnitines, phospholipids and the like), (iii) non-ionic surface-active agents, and (iv) mixtures of anionic surface-active agents (especially those having linear hydrocarbon regions) together with negative charge neutralizers. Negative charge neutralizers include but are not limited to acyl carnitines, cetyl pyridinium chloride, and the like. Acyl carnitines (e.g., lauroyl carnitine) are particularly good absorption enhancers. It is also preferred that the absorption enhancer is soluble at acid pH, particularly in the pH range of 3.0 to 5.0.

To reduce the likelihood of side effects, preferred surface-active agent of the invention, when used as the absorption enhancers of the invention, are either biodegradable or reabsorbable (e.g., biologically recyclable compounds such as bile acids, phospholipids, and/or acyl carnitines), preferably biodegradable. Acyl carnitines are believed particularly useful in enhancing paracellular transport.

Absorption enhancers may also include: (a) salicylates such as sodium salicylate, 3-methoxysalicylate, 5-methoxysalicylate and homovanilate; (b) bile acids such as taurocholic, tauorodeoxycholic, deoxycholic, cholic, glycholic, lithocholate, chenodeoxycholic, ursodeoxycholic, ursocholic, dehydrocholic, fusidic, etc.; (c) non-ionic surfactants such as polyoxyethylene ethers (e.g. Brij 36T, Brij 52, Brij 56, Brij 76, Brij 96, Texaphor A6, Texaphor A14, Texaphor A60 etc.), p-t-octyl phenol polyoxyethylenes (Triton X-45, Triton X-100, Triton X-114, Triton X-305 etc.) nonylphenoxypoloxyethylenes (e.g. Igepal CO series), polyoxyethylene sorbitan esters (e.g. Tween-20, Tween-80 etc.); (d) anionic surfactants such as dioctyl sodium sulfosuccinate; (e) lyso-phospholipids such as lysolecithin and lysophosphatidylethanolamine; (f) acylcarnitines, acylcholines and acyl amino acids such as lauroylcarnitine, myristoylcarnitine, palmitoylcarnitine, lauroylcholine, myristoylcholine, palmitoylcholine, hexadecyllysine, N-acylphenylalanine, N-acylglycine etc.; g) water soluble phospholipids such as diheptanoylphosphatidylcholine, dioctylphosphatidylcholine etc.; (h) medium-chain glycerides which are mixtures of mono-, di- and triglycerides containing medium-chain-length fatty acids (caprylic, capric and lauric acids); (i) ethylene-diaminetetraacetic acid; (j) cationic surfactants such as cetylpyridinium chloride; (k) fatty acid derivatives of polyethylene glycol such as Labrasol, Labrafac, etc.; and (I) alkylsaccharides such as lauryl maltoside, lauroyl sucrose, myristoyl sucrose, palmitoyl sucrose, etc.

In some preferred embodiments, and without intending to be bound by theory, cationic ion exchange agents (e.g., surface-active agent) are included to provide solubility enhancement by another possible mechanism. They may prevent the binding of the peptide active agents to the mucus. Preferred cationic ion exchange agents include protamine chloride or any other polycation.

Mixtures of absorption enhancers may also be used in the blend. Person skilled in the art would know which absorption enhancers may be blended together to provide preferable conditions for absorption of the peptide agents.

Preferably, the absorption enhancers is selected from acyl L-carnitine, preferably lauroyl L-carnitine.

### Organic acid

Organic acid is a component of the single dosage form of the invention comprised in the outer capsule.

The acid is believed to lower the local intestinal pH (where the active agent has been released) to levels below the optimal range for many intestinal proteases. Decrease in pH reduces the proteolytic activity of the intestinal proteases, thus affording protection to the peptide from potential degradation. The activity of these proteases is diminished by the temporarily acidic environment provided by the organic acid.

It is preferred that sufficient acid is provided that local intestinal pH is lowered temporarily to 5.5 or below, preferably 4.7 or below and more preferably 3.5 or below. Person skilled in the art would know how much organic acid should be used in the capsule to provide preferably pH. Preferably, conditions of reduced intestinal pH persist for a time period sufficient to protect the peptide agent from proteolytic degradation until at least some of the peptide agent has had an opportunity to cross the intestinal wall into the bloodstream. Absorption enhancers may synergistically promote peptide absorption into the blood while conditions of reduced proteolytic activity prevail. Preferred absorption enhancers were discussed above.

The organic acid of the invention is a pharmaceutically acceptable organic acid. Examples of the pharmaceutically acceptable acids include but are not limited to carboxylic acids such as acetylsalicylic, acetic, ascorbic, citric, fumaric, glucuronic, glutaric, glyoxylic, isocitric, isovaleric, lactic, maleic, oxaloacetic, propionic, pyruvic, succinic, tartaric, valeric, and the like, amino acids and salts thereof (e.g. amino acid hydrochlorides) or derivatives thereof such as acetylglutamic acid, alanine, arginine, asparagine, aspartic acid, carnosine, citrulline, creatine, glutamic acid, glycine, histidine, hydroxylysine, hydroxyproline, hypotaurine, isoleucine, leucine, lysine, methylhistidine, norleucine, ornithine, phenylalanine, proline, sarcosine, serine, taurine, threonine, tryptophan, tyrosine, valine, and the like.

Mixtures of organic acids may also be used. Person skilled in the art would know which organic acids may be blended together to provide preferable acidic conditions.

Preferably, the organic acid is citric acid.

### Other components

The single dosage form of the invention may optionally also include, in outer as well as in inner capsule, common pharmaceutical excipients. These typical for pharmaceutical formulations ingredients are not necessary for the invention but they should be treated as in the range of the invention.

Selection of the excipients is within the routine activities of one skilled in the art.

### Treatment

In accordance with the invention, patients in need of treatment with pharmaceutical peptide agent are provided with an oral pharmaceutical dosage form. The dosages and frequency of administering the products are discussed in more detail below. Patients who may benefit are any who suffer from disorders that respond favourably to increased levels of a peptide-containing compound.

It must be stressed that a kind of the treatment will depend on the type of peptide used in the single dosage form of the invention.

For example, single dosage form of the invention with Epithalon may be used for slowing the aging of the cell population by stimulating the renewal and elongation of telomeres, slowing down aging/exhibiting anti-aging effect, prevention of cancer and age-related diseases, restoration and normalization of the production of melatonin, strong acting as an antioxidant that protects against oxidative stress, or improvement of sleep, and its quality (for details, see: Khavinson VKh, Bondarev IE, Butyugov AA. Epithalon peptide induces telomerase activity and telomere elongation in human somatic cells. Bull Exp Biol Med. 2003 Jun;135(6):590-2. doi: 10.1023/a:1025493705728. PMID: 12937682; Goncharova ND, Khavinson BK, Lapin BA. Regulatory effect of Epithalon on production of melatonin and cortisol in old monkeys. Bull Exp Biol Med. 2001 Apr;131(4):394-6. doi: 10.1023/a:1017928925177. PMID: 11550036; Vanhee C, Moens G, Van Hoeck E, Deconinck E, De Beer JO. Identification of the small research tetra peptide Epitalon, assumed to be a potential treatment for cancer, old age and Retinitis Pigmentosa in two illegal pharmaceutical preparations. Drug Test Anal. 2015 Mar;7(3):259-64. doi: 10.1002/dta.1771. Epub 2014 Dec 22. PMID: 25535022.)

For example, single dosage form of the invention with GHK-Cu may be used for skin treatment/skin condition improvement, influencing lung function, Alzheimer's disease, and for gene regulation (for details, see Pickart, L., Vasquez-Soltero, J. M., & Margolina, A. (2015). GHK Peptide as a Natural Modulator of Multiple Cellular Pathways in Skin Regeneration. BioMed research international, 2015, 648108. https://doi.org/10.1155/2015/648108; Pickart L, Vasquez-Soltero JM, Margolina A. The human tripeptide GHK-Cu in prevention of oxidative stress and degenerative conditions of aging: implications for cognitive health. Oxid Med Cell Longev. 2012;2012:324832. doi:10.1155/2012/324832).

For example, single dosage form of the invention with BPC may be used for diseases, disorders or conditions for which neuroprotective effect, antidepressant effect, effect of alleviating diseases of the digestive system, help in the regeneration of the bones and joints, help with thrombosis, arterial hypertension, or help with skin diseases is necessary (for details, see: Gjurasin M, Miklic P, Zupancic B, Perovic D, Zarkovic K, Brcic L, Kolenc D, Radic B, Seiwerth S, Sikiric P. Peptide therapy with pentadecapeptide BPC 157 in traumatic nerve injury. Regul Pept. 2010 Feb 25;160(1-3):33-41. doi: 10.1016/j.regpep.2009.11.005. Epub 2009 Nov 10. PMID: 19903499; Knezevic, Mario et al. "Occluded Superior Mesenteric Artery and Vein. Therapy with the Stable Gastric Pentadecapeptide BPC 157." Biomedicines vol. 9,7 792. 8 Jul. 2021, doi:10.3390/biomedicines9070792; Hsieh, Ming-Jer et al. "Modulatory effects of BPC 157 on vasomotor tone and the activation of Src-Caveolin-1-endothelial nitric oxide synthase pathway." Scientific reports vol. 10,1 17078. 13 Oct. 2020, doi:10.1038/s41598-020-74022-y; Perovic, Darko et al. "Stable gastric pentadecapeptide BPC 157 can improve the healing course of spinal cord injury and lead to functional recovery in rats." Journal of orthopaedic surgery and research vol. 14,1 199. 2 Jul. 2019, doi:10.1186/s13018-019-1242-6; Takeo M, Lee W, Ito M. Wound healing and skin regeneration. Cold Spring Harb Perspect Med. 2015 Jan 5;5(1):a023267. doi: 10.1101/cshperspect.a023267. PMID: 25561722; PMCID: PMC4292081).

Because pharmaceutical dosage form for oral delivery of peptide agent provided by the invention protects the peptide active agents from proteolytic degradation and from the act of organic acid, it is expected to provide bioavailability of a wide range of therapeutic peptide active agents.

The amount of the peptide agent will be known by the person skilled in the art as a general knowledge or state of the art. The amount of the peptide agent should provide a therapeutic effect when delivered in the regime known in the art and specific for a king of peptide agent, and therefore should be in a range known in the art.

The amount of peptide agent and other ingredients is limited by the size of the capsules used. When higher amount of peptide agent is to be administered to a patient, it is obvious for the person skilled in the art that two or more single dosages forms may be administered at the same time.

The major advantage of the invention is to provide a single dosage form using commercially available capsules. The present approach does not require any special techniques or expensive equipment to manufacture the single dosage form as this was in the prior art, especially, in the case of coated acid particles. The single dosage form of the invention as a capsule-in-capsule system does not require tableting process, not to mention multilayer tableting. The approach of the invention separates spatially the peptide agent and the organic acid so that there is no contact between them at all. Even in the multilayer tablets of the prior art there it is still some contact causing degradation of the peptide agents.

As mentioned above, simple tableting itself carries many risks in relation to the manipulation of peptides in general. For example, contacting the peptide with water and to subjecting the peptide to drying steps at elevated temperature may degrade the peptide.

The simple single dosage form of the invention ensures a good bioavailability of the peptide agent.

The invention is further illustrated by the following non-limiting examples.

### Materials and methods

Enteric capsule of size 0, 00, 000 (made of pharmaceutical cellulose derivatives [HPMC-AS, HPMC]), enteric capsule of size 5, 4, 3 (made of pharmaceutical cellulose derivatives [HPMC-AS, HPMC]) or gelatin capsule (made of gelatine) was purchased from Capsugel.

Peptides was purchased from Lipopharm (http://www.lipopharm.pl/pl/)

Lauroyl L-carnitine, citric acid, and magnesium stearate were purchased from Sigma Aldrich Microcrystalline cellulose was purchased from Bart (https://bart.pl/substancje-pomocnicze/)

### Blood sampling and dosage form administration

A blood sampling was performed by collecting venous blood from the elbow vein at specific time points within the time range from 0 min to 360 min. The tests were performed on an empty stomach, taking only the test substance. The patient had to rest during the blood collection for the test. No sides effects of taking dosages such as drowsiness, nausea or general weakness were noticed. The test was performed after going through the correct daily cycle and following an appropriate, normal diet with typical nutrient content. HPLC measurements were carried out to indicate the concentration of the peptide agent. HPLC samples were prepared typically. To obtain serum, blood is collected in test tubes without an anticoagulant (blood sampling to a cloth tube). After the collection, the blood is allowed to stand for about 30-60 minutes. Spin - 5 minutes at 4000 rpm. The obtained serum should be drawn into a new, clean test tube.

### EXAMPLE 1

The single dosage form of the invention containing GHK-Cu peptide (Gly-His-Lys+Cu) was prepared by the manual technology. The peptide agent (20 mg) and lauroyl L-carnitine (1500 mg) were weighed out and blended.

Each enteric capsule of size 3 (inner capsule) was filled manually with 152 mg of the above blend so that the capsule contains 2 mg of the peptide agent and 150 mg lauroyl L-carnitine. The capsule was then closed.

500 mg of citric acid was introduced into the enteric capsule size 000 (outer capsule). Inner capsule was then placed in the outer capsule filled previously with the citric acid and the outer capsule was closed.

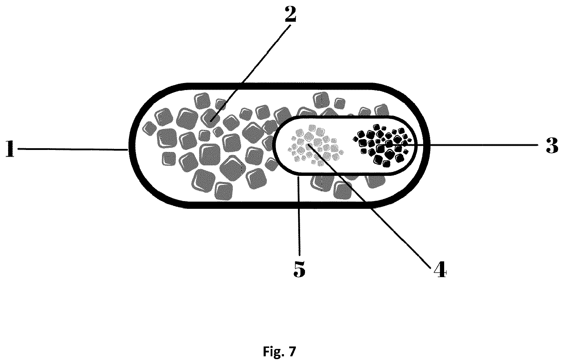

Schematic view of the single dosage form of the invention is shown in Fig. 7, where 5 is inner capsule comprising peptide agent 4 and absorption enhancer 3. Outer capsule 1 contains inner capsule 5 and organic acid 2.

### COMPARATIVE EXAMPLE 1.1

Comparative capsule was prepared by intermixing powdered 2 mg of GHK-Cu peptide, 150 mg of lauroyl L-carnitine and 500 mg of citric acid and placing them in the enteric capsule size 000.

### COMPARATIVE EXAMPLE 1.2

Comparative capsule was prepared by intermixing powdered 2 mg of GHK-Cu peptide, 800 mg microcrystalline cellulose and 200 mg of D-mannitol and placing them in the enteric capsule size 000.

### EXAMPLE 1.3

Blood test for EXAMPLE 1 and COMPARATIVE EXAMPLE 1.1, and 1.2

The single dosage form of the invention of EXAMPLE 1 as well as the single dosage form from COMPARATIVE EXAMPLE 1.1, and 1.2 were administered p.o. (orally) to a patient.

The plasma concentration of the peptide was studied in the range time from 0 min to 360 min after the swallowing of a capsule, wherein the 0 min time point was taken shortly after taking the capsule.

The peptide agent in the blood samples was quantified by high-performance liquid chromatography (HPLC) with the use of post-column ninhydrin derivatization and UV detection at the 220 nm, using standard curve method. The peptide concentration was calculated from the calibration curve in relation to the peptide standard purchased from Lipopharm.pl.

Liquid chromatograph Perkin-Elmer LC-200 with autosampler LC-200 and DIODE ARRAY LC-235 detector, TOTALCHROM ver. 6.3.2 software were used.

Column: Acclaim Mixed-Mode WAX-1 5 um (2,1 × 150mm), precolumn Acclaim Mixed-Mode WAX-1. Conditions of the HPLC run:
- Mobile phase flow: 0.2 mL/min
- Temperature: 35 °C
- Mobile phase:
A: 50 mM potassium phosphate
B: methanol
- Dosing loop - 10 µl
- Detector UV: 220 nm
- Gradient program:

| | |
|---|---|
| 98 % A | 5 min |
| 2 to 50 % B | 10 min |
| 50 % B | 5 min |

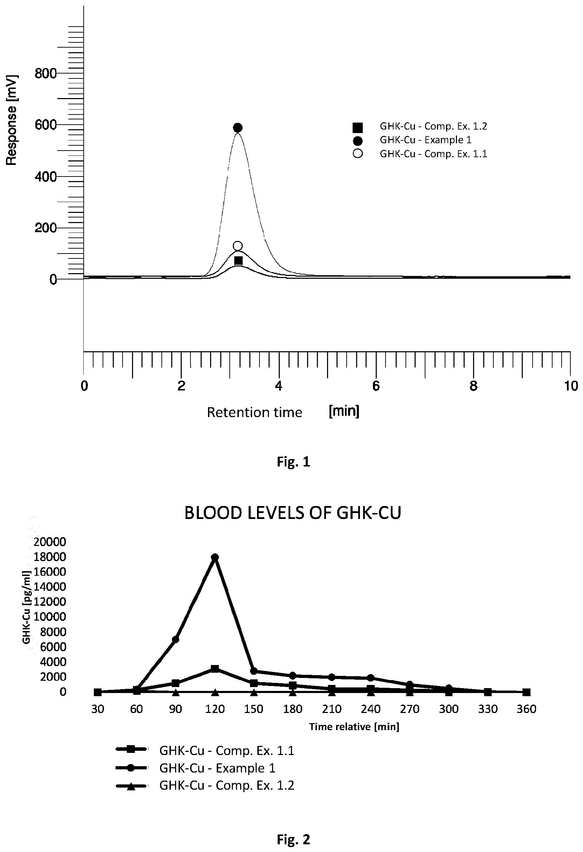

For comparative purposes, the peaks from HPLC analysis of: Example 1 (solid circle), Comp. Ex. 1.1 (open circle) and Comp. Ex. 1.2 (square) for the samples tested after 120 minutes were superimposed on one plot shown on Fig. 1.

Table 1 below shows the concentrations of the peptide agent calculated for Example 1, Comp. Ex. 1.1 and Comp. Ex. 1.2 shown at Fig. 1 (after 120 min).

**Table 1**

| Example | Peak # | Retention time [min] | Concentration [µg/sample] |
|---|---|---|---|
| GHK-Cu - Example 1 | 1 | 3.7 | 0.2 |
| GHK-Cu - Comp. Ex. 1.1 | 12 | 3.10 | 0.03 |
| GHK-Cu - Comp. Ex. 1.2 | 1 | 3.50 | 0 |

Corresponding data were collected for all samples taken in the time range from 0 min to 360 min. Fig. 2 shows plot of the concentrations of the peptide agent in the blood for Example 1 (circles), Comp. Ex. 1.1 (squares) and Comp. Ex. 1.2 (triangles). Three time points were chosen for detailed analysis: after 60, 90 and 120 min and concentrations in plasma are listed in the table 2 below:

**Table 2**

| Example | Peptide/Dosage | Peptide concentration in plasma [pg/ml] | | |
|---|---|---|---|---|
| | | 60 min | 90 min | 120 min |
| Example 1 | GHK-Cu/of the invention | 350 | 7000 | 18000 |
| Comparative Example 1.1 | GHK-Cu/the same composition as of the invention, but one enteric capsule | 320 | 1200 | 3100 |
| Comp. Example 1.2 | GHK-Cu/cellulose, D-mannitol, in one enteric capsule | 150 | 0 | 0 |

Fig. 2 shows graphically the pharmacokinetic profiles for the peptide agent GHK-Cu quantified in the blood in the specific time range for Example 1, Comp. Ex. 1.1 and Comp. Ex. 1.2.

After 60 minutes, the concentration of GHK-Cu for Example 1 is 350 pg/ml, whereas for the same composition as of the invention, but intermixed in one simple enteric capsule (Comp. Ex. 1.1.) is 320 pq/ml, and for a mixture with typical ingredients closed in one simple enteric capsule is 150 pg/ml. For all tested single dosage forms, 60 minutes after taking the dosage a significant concentration of the tested peptide agent in the blood is revealed.

After 90 minutes, the concentration of GHK-Cu for the Example 1 is much higher than for comparative examples. After 120 minutes, the differences are more pronounces, and the concentration of GHK-Cu for Example 1 is 18000 pq/ml, for Comp. Ex. 1.1 is 3100 pq/ml, whereas for Comp. Ex. 1.2 is 0 pg/ml.

The highest values as shown on Fig. 2 were obtained after 120 minutes for all tested single dosage forms, but for Example 1 the concentration of the peptide agent in the blood was almost 6 times higher than for Comp. Ex. 1.1., where citric acid, the absorption enhancer and the peptide agent were in the same compartment intermixed in one capsule.

### EXAMPLE 2

The single dosage form of the invention containing Epithalon peptide (Ala-Glu-Asp-Gly) was prepared by the manual technology. The peptide agent (20 mg) and lauroyl L-carnitine (1000 mg) were weighed out and blended.

Each enteric capsule of size 4 (inner capsule) was filled manually with 102 mg of the above blend so that the capsule contains 2 mg of the peptide agent and 100 mg lauroyl L-carnitine. The capsule was then closed.

400 mg of citric acid was introduced into the enteric capsule size 00 (outer capsule). Inner capsule was then placed in the outer capsule filled previously with the citric acid and the outer capsule was closed.

### COMPARATIVE EXAMPLE 2.1

Comparative capsule was prepared by intermixing powdered 2 mg of Epithalon peptide, 100 mg of acyl L-carnitine and 400 mg of citric acid and placing them in the enteric capsule size 00.

### COMPARATIVE EXAMPLE 2.2

Comparative capsule was prepared by intermixing powdered 2 mg of Epithalon peptide, 500 mg cellulose and 50 mg of magnesium stearate and placing them in the enteric capsule size 00.

### EXAMPLE 2.3

Blood test for EXAMPLE 2 and COMPARATIVE EXAMPLE 2.1, and 2.2

The single dosage form of the invention of EXAMPLE 2 as well as the single dosage form from COMPARATIVE EXAMPLE 2.1, and 2.2 were administered p.o. (orally) to a patient.

The plasma concentration of the peptide was studied in the range time from 0 min to 360 min after the swallowing of a capsule, wherein the 0 min time point was taken shortly after taking the capsule.

The peptide agent in the blood samples was quantified by high-performance liquid chromatography (HPLC) with the use of post-column ninhydrin derivatization and UV detection at the 220 nm, using standard curve method.

Liquid chromatograph Perkin-Elmer LC-200 with autosampler LC-200 and DIODE ARRAY LC-235 detector, TOTALCHROM ver. 6.3.2 software were used.

Column: Acclaim Mixed-Mode WAX-1 5 um (2,1 × 150mm), precolumn Acclaim Mixed-Mode WAX-1.

Conditions of the HPLC run:
- Mobile phase flow: 0.2 mL/min
- Temperature: 35 °C
- Mobile phase:
A: 50 mM potassium phosphate
B: methanol
- Dosing loop - 10 µl
- Detector UV: 220 nm
- Gradient program:

| | |
|---|---|
| 98 % A | 5 min |
| 2 to 50 % B | 10 min |
| 50 % B | 5 min |

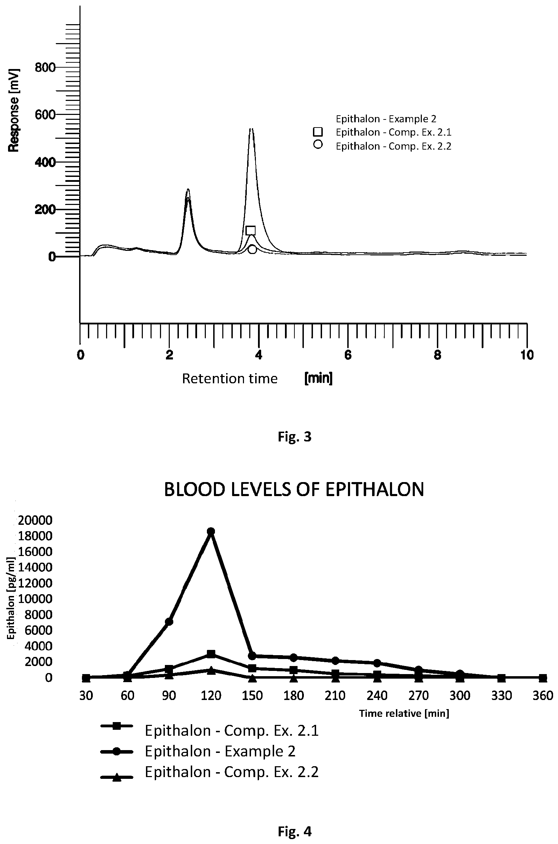

For comparative purposes, the peaks from HPLC analysis of: Example 2 (solid circle), Comp. Ex. 2.1 (square) and Comp. Ex. 2.2 (open circle) for the samples tested after 120 minutes were superimposed on one plot shown on Fig. 3.

Table 3 below shows the concentrations of the peptide agent calculated for Example 2, Comp. Ex. 2.1 and Comp. Ex. 2.2 shown at Fig. 3 (after 120 min).

**Table 3**

| Example | Peak # | Retention time [min] | Concentration [µg/sample] |
|---|---|---|---|
| Epithalon - Example 2 | 2 | 3.82 | 0.19 |
| Epithalon - Comp. Ex. 2.1 | 2 | 3.9 | 0.028 |
| Epithalon - Comp. Ex. 2.2 | 2 | 3.534 | 0 |

Corresponding data were collected for all samples taken in the time range from 0 min to 360 min. Fig. 4 shows plot of the concentrations of the peptide agent in the blood for Example 2 (circles), Comp. Ex. 2.1 (squares) and Comp. Ex. 2.2 (triangles). Three time points were chosen for detailed analysis: after 60, 90 and 120 min and concentrations in plasma are listed in the table 4 below:

**Table 4**

| Example | Peptide/Dosage | Peptide concentration in plasma [pg/ml] | | |
|---|---|---|---|---|
| | | 60 min | 90 min | 120 min |
| Example 2 | Epithalon/of the invention | 360 | 7150 | 18600 |
| Comparative Example 2.1 | Epithalon/the same composition as of the invention, but one enteric capsule | 310 | 1150 | 3000 |
| Comp. Example 2.2 | Epithalon/microcrystalline cellulose, magnesium stearate in one enteric capsule | 50 | 400 | 1000 |

Fig. 4 shows graphically the pharmacokinetic profiles for the peptide agent Epithalon quantified in the blood in the specific time range for Example 2, Comp. Ex. 2.1 and Comp. Ex. 2.2.

After 60 minutes, the concentration of Epithalon for Example 2 is 360 pg/ml, whereas for the same composition as of the invention, but intermixed in one simple enteric capsule (Comp. Ex. 2.1.) is 310 pq/ml, and for a mixture with typical ingredients closed in one simple enteric capsule is 50 pg/ml. For all tested single dosage forms, 60 minutes after taking the dosage a significant concentration of the tested peptide agent in the blood is revealed.

After 90 minutes, the concentration of Epithalon for the Example 2 is much higher than for comparative examples. After 120 minutes, the differences are more pronounces, and the concentration of Epithalon for Example 2 is 18600 pq/ml, for Comp. Ex. 2.1 is 3000 pq/ml, whereas for Comp. Ex. 2.2 is 1000 pg/ml.

The highest values as shown on Fig. 4 were obtained after 120 minutes for all tested single dosage forms, but for Example 2 the concentration of the peptide agent in the blood was over 6 times higher than for Comp. Ex. 2.1., where citric acid, the absorption enhancer and the peptide agent were in the same compartment intermixed in one capsule.

### EXAMPLE 3

The single dosage form of the invention containing Arg-BPC-157 peptide (Gly-Glu-Pro-Pro-Pro-Gly-Lys-Pro-Ala-Asp-Asp-Ala-Gly-Leu-Val-Arg) was prepared by the manual technology. The peptide agent (20 mg) and lauroyl L-carnitine (500 mg) were weighed out and blended.

Each enteric capsule of size 5 (inner capsule) was filled manually with 52 mg of the above blend so that the capsule contains 2 mg of the peptide agent and 50 mg lauroyl L-carnitine. The capsule was then closed.

300 mg of citric acid was introduced into the enteric capsule size 0 (outer capsule). Inner capsule was then placed in the outer capsule filled previously with the citric acid and the outer capsule was closed.

### COMPARATIVE EXAMPLE 3.1

Comparative capsule was prepared by intermixing powdered 2 mg of Arg-BPC-157 peptide, 50 mg of acyl L-carnitine and 300 mg of citric acid and placing them in the enteric capsule size 0.

### COMPARATIVE EXAMPLE 3.2

Comparative capsule was prepared by intermixing powdered 2 mg of Arg-BPC-157 peptide, 300 mg microcrystalline cellulose and 50 mg of magnesium stearate and placing them in the enteric capsule size 0.

### EXAMPLE 3.3

Blood test for EXAMPLE 3 and COMPARATIVE EXAMPLE 3.1, and 3.2

The single dosage form of the invention of EXAMPLE 3 as well as the single dosage form from COMPARATIVE EXAMPLE 3.1, and 3.2 were administered p.o. (orally) to a patient.

The plasma concentration of the peptide was studied in the range time from 0 min to 360 min after the swallowing of a capsule, wherein the 0 min time point was taken shortly after taking the capsule.

The peptide agent in the blood samples was quantified by high-performance liquid chromatography (HPLC) with the use of post-column ninhydrin derivatization and UV detection at the 215 nm, using standard curve method.

Liquid chromatograph Perkin-Elmer LC-200 with autosampler LC-200 and DIODE ARRAY LC-235 detector, TOTALCHROM ver. 6.3.2 software were used.

Column: Waters HPLC Column Xselect Peptide CSH C18 130 A 3.5 um (4.6 x 100mm).

Conditions of the HPLC run:
- Mobile phase flow: 0.2 mL/min
- Temperature: 40 °C
- Mobile phase:
A: 0.1% formic acid in 100% water
B: 0.085% formic acid in 75%/25% acetonitrile/water
- Dosing loop - 20 µl
- Detector UV: 215 nm
- Gradient program:

| | |
|---|---|
| 1 to 21% B | 5 min |
| 21 to 31.4% B | 2.5 min |
| 31.4 to 95% B | 2.5 min |
| 95% B | 1.5 min |
| 1% B | 2.5 min |

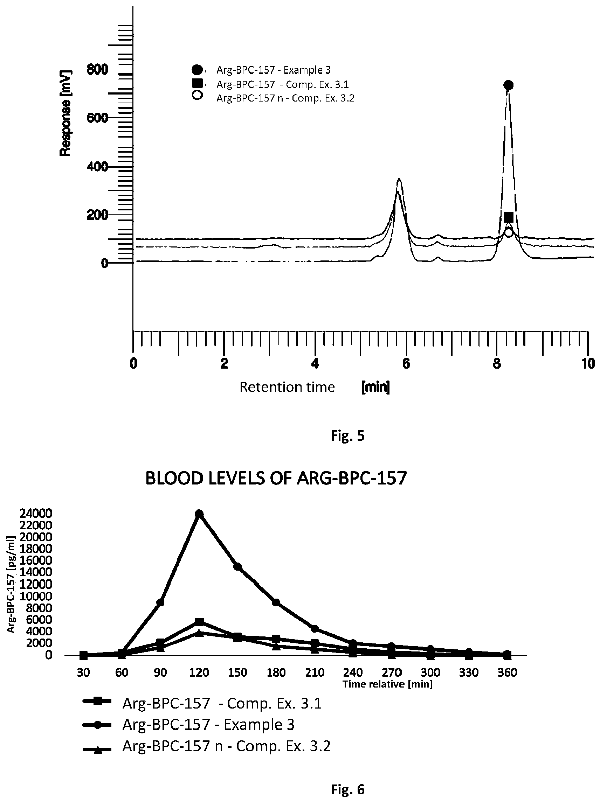

For comparative purposes, the peaks from HPLC analysis of: Example 3 (solid circle), Comp. Ex. 3.1 (square) and Comp. Ex. 3.2 (open circle) for the samples tested after 120 minutes were superimposed on one plot shown on Fig. 5.

Table 5 below shows the concentrations of the peptide agent calculated for Example 3, Comp. Ex. 3.1 and Comp. Ex. 3.2 shown at Fig. 5 (after 120 min).

**Table 5**

| Example | Peak # | Retention time [min] | Concentration [µg/sample] |
|---|---|---|---|
| Arg-BPC-157 - Example 3 | 3 | 8.2 | 0.28 |
| Arg-BPC-157 - Comp. Ex. 3.1 | 3 | 8.24 | 0.042 |
| Arg-BPC-157 - Comp. Ex. 3.2 | 3 | 8.34 | 0 |

Corresponding data were collected for all samples taken in the time range from 0 min to 360 min. Fig. 6 shows plot of the concentrations of the peptide agent in the blood for Example 3 (circles), Comp. Ex. 3.1 (squares) and Comp. Ex. 3.2 (triangles). Three time points were chosen for detailed analysis: after 60, 90 and 120 min and concentrations in plasma are listed in the table 6 below:

**Table 6**

| Example | Peptide/Dosage | Peptide concentration in plasma [pg/ml] | | |
|---|---|---|---|---|
| | | 60 min | 90 min | 120 min |
| Example 3 | Arg-BPC-157/ of the invention | 420 | 9000 | 24000 |
| Comparative Example 3.1 | Arg-BPC-157/ the same composition as of the invention, but one enteric capsule | 410 | 2100 | 5700 |
| Comp. Example 3.2 | Arg-BPC-157/ cellulose, magnesium stearate, in one enteric capsule | 120 | 1300 | 3800 |

Fig. 6 shows graphically the pharmacokinetic profiles for the peptide agent Arg-BPC-157 quantified in the blood in the specific time range for Example 3, Comp. Ex. 3.1 and Comp. Ex. 3.2.

After 60 minutes, the concentration of Arg-BPC-157 for Example 3 is 420 pg/ml, whereas for the same composition as of the invention, but intermixed in one simple enteric capsule (Comp. Ex. 3.1.) is 410 pq/ml, and for a mixture with typical ingredients closed in one simple enteric capsule is 120 pg/ml. For all tested single dosage forms, 60 minutes after taking the dosage a significant concentration of the tested peptide agent in the blood is revealed.

After 90 minutes, the concentration of Arg-BPC-157 for the Example 3 is much higher than for comparative examples. After 120 minutes, the differences are more pronounces, and the concentration of Arg-BPC-157 for Example 3 is 24000 pq/ml, for Comp. Ex. 3.1 is 5700 pq/ml, whereas for Comp. Ex. 3.2 is 3800 pg/ml.

The highest values as shown on Fig. 6 were obtained after 120 minutes for all tested single dosage forms, but for Example 3 the concentration of the peptide agent in the blood was over 4 times higher than for Comp. Ex. 3.1., where citric acid, the absorption enhancer and the peptide agent were in the same compartment intermixed in one capsule.

The above examples show the better absorption of the peptide agent in the single dosage for according to the invention compared with the dosages known in at.

## Claims

1. A pharmaceutical single dosage form for oral delivery consisting of two capsules, an outer enteric capsule, and an inner capsule, wherein said inner capsule comprises at least one physiologically active peptide agent and at least one absorption enhancer, and wherein said outer capsule contains inner capsule and an organic acid.

2. The pharmaceutical single dosage form according to claim 1 wherein the peptide agent is composed of 3-20 amino acids, preferably 3-16.

3. The pharmaceutical single dosage form according to claim 2 wherein the peptide agent is selected from GHK-Cu, Epithalon, ARG-BPC-157.

4. The pharmaceutical single dosage form according to claim 1 or 2 wherein the inner capsule is enteric capsule or non-enteric capsule.

5. The pharmaceutical single dosage form according to any proceeding claim wherein absorption enhancer is selected from the group comprises (a) salicylates such as sodium salicylate, 3-methoxysalicylate, 5-methoxysalicylate and homovanilate; (b) bile acids such as taurocholic, tauorodeoxycholic, deoxycholic, cholic, glycholic, lithocholate, chenodeoxycholic, ursodeoxycholic, ursocholic, dehydrocholic, fusidic, etc.; (c) non-ionic surfactants such as polyoxyethylene ethers (e.g. Brij 36T, Brij 52, Brij 56, Brij 76, Brij 96, Texaphor A6, Texaphor A14, Texaphor A60 etc.), p-t-octyl phenol polyoxyethylenes (Triton X-45, Triton X-100, Triton X-114, Triton X-305 etc.) nonylphenoxypoloxyethylenes (e.g. Igepal CO series), polyoxyethylene sorbitan esters (e.g. Tween-20, Tween-80 etc.); (d) anionic surfactants such as dioctyl sodium sulfosuccinate; (e) lyso-phospholipids such as lysolecithin and lysophosphatidylethanolamine; (f) acylcarnitines, acylcholines and acyl amino acids such as lauroylcarnitine, myristoylcarnitine, palmitoylcarnitine, lauroylcholine, myristoylcholine, palmitoylcholine, hexadecyllysine, N-acylphenylalanine, N-acylglycine etc.; g) water soluble phospholipids such as diheptanoylphosphatidylcholine, dioctylphosphatidylcholine etc.; (h) medium-chain glycerides which are mixtures of mono-, di- and triglycerides containing medium-chain-length fatty acids (caprylic, capric and lauric acids); (i) ethylene-diaminetetraacetic acid; (j) cationic surfactants such as cetylpyridinium chloride; (k) fatty acid derivatives of polyethylene glycol such as Labrasol, Labrafac, etc.; and (I) alkylsaccharides such as lauryl maltoside, lauroyl sucrose, myristoyl sucrose, palmitoyl sucrose; cationic ion exchange agents (e.g. surface-active agent) such as protamine chloride or any other polycation, and their mixtures.

6. The pharmaceutical single dosage form according to claim 5 wherein absorption enhancer is acyl L-carnitine, preferably lauroyl L-carnitine.

7. The pharmaceutical single dosage form according to any proceeding claim wherein organic acid is selected from the group comprises carboxylic acids such as acetylsalicylic, acetic, ascorbic, citric, fumaric, glucuronic, glutaric, glyoxylic, isocitric, isovaleric, lactic, maleic, oxaloacetic, propionic, pyruvic, succinic, tartaric, valeric, and the like, amino acids and salts thereof (e.g. amino acid hydrochlorides) or derivatives thereof such as acetylglutamic acid, alanine, arginine, asparagine, aspartic acid, betaine, carnitine, carnosine, citrulline, creatine, glutamic acid, glycine, histidine, hydroxylysine, hydroxyproline, hypotaurine, isoleucine, leucine, lysine, methylhistidine, norleucine, ornithine, phenylalanine, proline, sarcosine, serine, taurine, threonine, tryptophan, tyrosine, valine, and their mixtures.

8. The pharmaceutical single dosage form according to claim 7 wherein organic acid is citric acid.

9. The pharmaceutical single dosage form according to claim 1-8 wherein the amount of peptide agent is 0.2 mg - 20 mg, the amount of absorption enhancer is 50 mg - 150 mg and the amount of organic acid is 200 mg - 500 mg per single dosage form.
